(19) Europäisches Patentamt
European Patent Office
Office européen des brevets

(11) Publication number: 0 128 721 B1

(12) **EUROPEAN PATENT SPECIFICATION**

(45) Date of publication of patent specification: **26.07.89**

(51) Int. Cl.$^4$: **B 01 J 29/06,** C 07 C 15/02, C 07 C 6/12, C 07 C 2/66, C 07 C 2/86

(21) Application number: **84303751.6**

(22) Date of filing: **04.06.84**

(54) **Silica-modified catalyst and use for selective production of para-dialkyl substituted benzenes.**

(30) Priority: **09.06.83 US 502856**
**09.06.83 US 502868**

(43) Date of publication of application:
**19.12.84 Bulletin 84/51**

(45) Publication of the grant of the patent:
**26.07.89 Bulletin 89/30**

(84) Designated Contracting States:
**BE DE FR GB IT NL**

(56) References cited:
**EP-A-0 082 019**
**US-A-3 980 586**
**US-A-4 379 761**
**US-A-4 402 867**

(73) Proprietor: **MOBIL OIL CORPORATION**
**150 East 42nd Street**
**New York New York 10017 (US)**

(72) Inventor: **Rodewald, Paul Gerhard**
**4 Merritt Lane**
**Rocky Hill New Jersey 08553 (US)**

(74) Representative: **Carpmael, John William Maurice et al**
**CARPMAELS & RANSFORD 43 Bloomsbury Square**
**London, WC1A 2RA (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).



Courier Press, Leamington Spa, England.

**Description**

This invention relates to a zeolite composition suitable as a catalyst for the selective production of para-dialkyl substituted benzenes and to a process for converting specified chargestocks to a high yield of para-dialkyl substituted benzenes utilizing such catalyst.

The disproportionation of aromatic hydrocarbons in the presence of zeolite catalysts has been described by Grandio et al. in the Oil and Gas Journal, Vol. 69, No. 48 (1971). U.S. Patent Nos. 3,126,422; 3,413,374; 3,598,878; 3,598,879 and 3,607,961 show vapor-phase disproportionation of toulene over various catalysts.

In these prior art processes, the xylene product produced has the equilibrium composition of approximately 24 percent para, 54 percent meta and 22 percent ortho. Of the xylene isomers, i.e. ortho, meta and para-xylene, meta-xylene is the least desired product, with para-xylene being the most desired product. Para-xylene is of particular value being useful in the manufacture of terephthalic acid which is an intermediate in the manufacture of synthetic fibers, such as "Dacron". Mixtures of xylene isomers, either alone or in further admixture with ethylbenzene, generally containing a concentration of about 24 weight percent para-xylene in the equilibrium mixture, have previously been separated by expensive super-fractionation and multistage refrigeration steps. Such process, as will be realized, has involved high operation costs and has a limited yield.

In addition to the above patents, other related prior art includes U.S. Patent No. 2,904,607 which refers to alkylation of aromatic hydrocarbons with an olefin in the presence of a crystalline metallic aluminosilicate having uniform pore openings of about 6 to 15 Angstrom units. U.S. Patent No. 3,251,897 describes alkylation of aromatic hydrocarbons in the presence of X- or Y-type crystalline alumuniosilicate zeolites, specifically such type zeolites wherein the cation is rare earth and/or hydrogen. U.S. Patent Nos. 3,751,504 and 3,751,506 describes vapor phase alkylation of aromatic hydrocarbons with olefins, e.g. benzene with ethylene, in the presence of a ZSM-5 type zeolite catalyst.

The alkylation of toluene with methanol in the presence of a cation exchanged zeolite Y has been described by Yashima et al. in the Journal of Catalysis 16, 273—280 (1970). The workers reported selective production of paraxyelen over the approximate temperature range of 200° to 275°C, with the maximum yield of para-xylene in the mixture of xylenes, i.e. about 50 percent of the xylene product mixture being observed at 225°C. Higher temperatures were reported to result in an increase in the yield of metal-xylene and a decrease in the production of para and ortho-xylenes. U.S. patent No. 3,965,210 describes alkylation of toluene with methanol in the presence of a crystalline aluminosilicate zeolite, such as ZSM-5, which has been modified by contact with a polymer made up of meta-carborane units connected by siloxane units to selectively yield para-xylene. These latter catalysts have, however, suffered from the serious deficiency of loss of selectivity upon air regeneration. This is attributable to breakage of carbon-silicon bonds upon exposure to the high temperature of regeneration giving rise to isolated clusters of silica on the zeolite surface rather than the extensive surface coverage afforded by the technique decribed herein.

U.S. Patent No. 2,722,504 describes a catalyst of an activated oxide such as silica gel having a thin layer of a silicone polymer deposited thereon to increase the oranophilic character of the contact surface and, as such, seeks to avoid silica deposition.

Crystalline aluminosilicate zeolites, modified by reaction with an organic substituted silane, have been described in U.S. Patent Nos. 3,682,996 and 3,698,157. The former of these patents describes, as novel compositions of matter, crystalline aluminosilicate esters made by reacting a crystalline aluminosilicate having an available hydrogen atom with an organic silane having a SiH group. The resulting compositions were disclosed as being catalysts useful for hydrocarbon processes, particularly hydrocracking. In the latter of the above patents, the use of ZSM-5 type crystalline aluminosilicate zeolites modified by treatment with an organic-radical substituted silane are described, together with the use of such modified zeolites in chromatographic separation of the compounds in a $C_8$ aromatic feed stock.

U.S. Patent No. 4,145,315 discloses a method for the production of silica modified zeolite catalysts which are prepared by contacting the specific zeolite with an organic solvent solution such as hexane, of a silicone fluid, distillation of the hexane, and air calcination of the zeolite residue.

Silica-modified catalysts are shown in U.S. Patent Nos. 4,379,761; 4,100,219 and 4,090,981. In each instance the silica modification results from interaction of the zeolite portion of the catalyst with an organic solution comprising a silica source such as a silicone.

U.S. Patent No. 4,088,605 shows alteration of a crystallisation medium to substantially eliminate aluminum during crystallization in order to synthesize a zeolite with a coating of silica.

In accordance with the present invention, there has been discovered a method for preparing a zeolite which is particularly applicable for the selective conversion of aromatic feedstocks to conversion products rich in para-dialkyl substituted benzenes. Such a method comprises the steps of contacting a crystalline zeolite base components of particularly defined activity and xylene sorption characteristics with an aqueous emulsion or dispersion of a particular type of silicon-containing compound, e.g. a silicone, and by heating this crystalline zeolite base component, which has been treated with the silicon-containing compound, in an oxygen-containing atmosphere at a temperature of from 300°C to 700°C.

The zeolite base component is one which has an alpha activity of from 2 to 5000, a xylene sorption capacity greater than 1 gram xylene per 100 grams of zeolite, an ortho-xylene sorption time for 30 percent

of said sorption capacity measured at 120°C and a xylene partial pressure of 600 ± 100 Pa (4.5 to 0.8 mm Hg) of greater than 10 minutes, a $SiO_2/Al_2O_3$ mole ratio of at least 12, a constraint index within the approximative range of 12 mol, after heating at 540°C for 1 hour in an inert atmosphere, an intercrystalline capacity for normal hexane which is greater than that for water.

The silicon-containing compound used in the emulsion or dispersion which contacts the zeolite base component is of a molecular size substantially incapable of entering the pores of zeolite. Such a silicon-containing compound has the general formula:

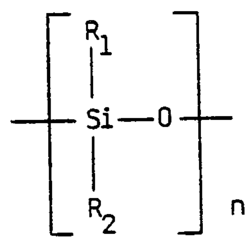

wherein

$R_1$ is hydrogen, fluorine, hydroxy, alkyl, aralkyl, alkaryl or fluoro-alkyl, the hydrogen substituents containing from 1 to 10 carbon atoms;

$R_2$ is selected from the same group as $R_1$, other than hydrogen, and other than methyl if $R_1$ is hydrogen; and

n is an integer of at least 10.

Contact of zeolitie base component with silicon-containing compound emulsion or dispersion occurs at a temperature of from 10°C to 200°C for a time sufficient to permit from 0.5 weight percent to 30 weight percent silica to be deposited on the crystalline zeolite base component when the treated base component is heated in an oxygen-containing atmosphere at a temperature of from 300°C to 700°C.

The resulting zeolite composition has deposited thereon a coating of silica which extensively covers and resides substantially exclusively on the external surface of the zeolite composition. The resulting zeolite composition has been found to possess a long catalytic life, e.g. to be capable of regeneration after catalytic use without substantial loss in activity.

Catalysts containing the zeolite compositions hereinbefore described has been found to be particularly useful in the selective production of para-dialkyl substituted benzenes containing alkyl groups of 1 to 4 carbon atoms, such as para-xylene, by conversion in the presence thereof, of a hydrocarbon precursor such as a mono alkyl-substituted benzene having 1—4 carbon atoms in the alkyl substituent or a mixture of such precursor or benzene with an alkylating agent containing from 1 to 4 carbon atoms. Typical of such conversion processes are the disproportionation of toluene and the alkylation of benzene or toluene with a methylating agent, e.g. methanol.

In a preferred embodiment, catalysts employing the zeolite composition prepared by the present method can be used in a process which comprises conversion of the specified aromatic precursor reactants to yield xylenes in which the proportion of para-xylene is substantially in excess of the normal equilibrium concentration and preferably in excess of 30 weight percent of the xylene product produced. Such conversion can be carried out in the presence of such catalysts at a temperature between about 250°C and about 750°C and a pressure between about 101.3 kPa to 10133 kPa (0.1 and about 100 atmospheres), utilizing a feed weight hourly space velocity (WHSV) between about 0.1 and about 2000. The latter WHSV is based upon the weight of catalyst composition, i.e. total weight of active zeolite composition plus binder therefor. The effluent can be separated and distilled to remove the desired product, e.g. para-xylene, and unreacted material is recycled for further reaction.

As noted hereinbefore, the zeolite base component of the present catalyst upon which silica deposition is effected is characterized by particular activity and sorption properties. Thus, the porous crystalline aluminosilicate zeolite employed herein necessarily has: (1) an activity, in terms of alpha value, of between about 2 and about 5000, (2) a xylene sorption capacity greater than 1 gram/100 grams of zeolite and (3) an ortho-xylene sorption time for 30 percent of said capacity of greater than 10 minutes, where the sorption capacity and sorption time are measured at 120°C and a xylene of 4.5 ± 0.8 mm of mercury (600 ± 100 Pa).

The alpha value reflects the relative activity of the catalyst with respect to a high activity silica-alumina cracking catalyst. To determine the alpha value as such term is used herein, n-hexane conversion is determined at about 538°C. Conversion is varied by variation in space velocity such that a conversion level of 10 to 60 percent of n-hexane is obtained and converted to a rate constant per unit volume of zeolite and compared with that of silica-alumina catalyst which is normalized to a reference activity of 538°C. Catalytic activity of the catalysts are expressed as multiple of this standard, i.e. the silica-alumina standard. The silica-alumina reference catalyst contains about 10 weight percent $Al_2O_3$ and remainder $SiO_2$. This method of determining alpha, modified as described above, is more fully described in the *Journal of Catalysis, Vol. VI*, Pages 278—287, 1966, as well as in U.S. Patent No. 3,354,078.

The measurements of hydrocarbon sorption capacities and rates are conveniently carried out gravimetrically in a thermal balance. In particular, it has been found that an equilbrium sorption capacity of xylene, which can be either para, meta, ortho or a mixture thereof, preferably para-xylene since this isomer reaches equilibrium within the shortest time of at least 1 gram per 100 grams of zeolite measured at 120°C and xylene partial pressure of 4.5 ± 0.8 mm of mercury (600 ± 100 Pa) and an orthoxylene soprtion time for 30 percent of said capacity of greater than 10 minutes (at the same conditions of temperature and pressure) are required in order to achieve the desired selective production of para-dialkyl substituted benzenes.

It has been found that zeolites exhibiting very high selectivity for para-dialkylbenzene production require a very long time up to an exceeding a thousand minutes to sorb o-xylene in an amount of 30% of total xylene soprtion capacity. For those materials it is more convenient to determine the sorption time for a lower extent of sorption, such as 5%, 10%, or 20% of capacity, and to estimate the 30% sorption time by applying the following multiplication factors F as illustrated for 5% sorption:

| $t_{0.3} = F.t_{0.05}$ Percent of sorption capacity | Factor (F) to Estimate 30% Sorption Time |
|---|---|
| 5 | 36 |
| 10 | 9 |
| 20 | 2.2 |

Zeolites within the confines of this invention have a silica to alumina ratio of at least about 12 and a constant index within the approximate range of 1 to 12. These zeolites induce profound transformation of aliphatic hydrocarbons to aromatic hydrocarbon in commercially desirable yields and are generally highly effective in conversion reactions involving aromatic hydrocarbons. Although they have usually low alumina contents, i.e. high silica to alumina ratios, they are very active even when the silica to alumina ratio exceeds 30. The activity is surprising since catalytic activity is generally attributed to framework aluminum atoms and cations associated with these aluminum atoms. These zeolites retain their crystallinity for long periods in spite of the presence of steam at high temperature which induces irreversible collapse of the framework of other zeolites e.g. of the X and A type. Furthermore, carbonaceous deposits, when formed, may be removed by burning at higher than usual temperatures to restore activity. In many environments the zeolites of this class exhibit very low coke forming capability, conducive to very long times on stream between burning regenerations.

An important characteristic of the crystal structure of this preferred class of zeolites is that it provides constrained access to, and egress from the intracrystalline free space by virtue of having a pore dimension greater than about 5 Anstroms and pore windows of about a size such as would be provided by 10-membered rings of oxygen atoms. It is to be understood, of course, that these rings are those formed by the regular disposition of the tetrahedra making up the anionic framework of the crystalline aluminosilicate, the oxygen atoms themselves being bonded to the silicon or aluminum atoms at the centres of the tetrahedra. Briefly, the preferred type zeolites useful in this invention possess, in combination: a silica to alumina mole ratio of at least about 12; and a structure providing constrained access to the crystalline free space as measured by having a constraint index of from 1 to 12.

Zeolite materials which have a silica to alumina molar ratio of at least 12 and a constraint index within the range of 1 to 12 are well known. Such zeolites and their use as catalysts for the conversion of aromatics are generally described, for example, in the aforementioned U.S. Patent No. 4,379,761. Crystalline zeolites of the preferred type useful as the base zolite component in the present invention include ZSM—5, SZM—5/ZSM—11 intermediate, ZSM—11, ZSM—12, ZSM—23, ZSM—35, ZSM—38, and ZSM—48, with ZSM—5 being particularly preferred.

ZSM—5 is described in greater detail in U.S. Patent Nos. 3,702,883 and RE 29,948, which patents provide the X-ray diffraction pattern of the therein disclosed ZSM—5.

ZSM—11 is described in U.S. Patent No. 3,709,979, which discloses in particular the X-ray diffraction pattern of ZSM—11.

ZSM—5/ZSM—11 intermediate is described in U.S. Patent No. 4,229,424, which discloses in particular the X-ray diffraction pattern of ZSM—5/ZSM—11 intermediate.

ZSM—12 is described in U.S. Patent No. 3,832,449, which discloses in particular the X-ray diffraction pattern of ZSM—12.

ZSM—23 is described in U.S. Patent No. 4,076,842, which discloses in particular the X-ray diffraction pattern of ZSM—23.

ZSM—35 is described in U.S. Patent No. 4,016,245, which discloses in particular the X-ray diffraction pattern of ZSM—35.

ZSM—38 is described in U.S. Patent No. 4,046,859, which discloses in particular the X-ray diffraction pattern of ZSM—38.

ZSM—48 is described in U.S. Patent No. 4,375,573, which discloses in particular the X-ray diffraction

pattern of ZSM—48.

When synthesized in the alkali metal form, the zeolite base component is conveniently converted to the hydrogen form, generally by intermediate formation of the ammonium form as a result of ammonium ion exchange and calcination of the ammonium form to yield the hydrogen form. In addition to the hydrogen form, other forms of the zeolite wherein the original alkali metal has been reduced to less than about 1.5 percent by weight, and often to less than about 0.5 percent by weight, may be used. Thus, the original alkali metal of the zeolite may be replaced by ion exchange with other suitable ions of Groups IB to VIII of the Periodic Table, including, by way of example, nickel, copper, zinc, palladium, platinum, calcium or rare earth metals.

In practicing the selective conversion process of the present invention, it may be desirable to incorporate the above described crystalline aluminosilicate zeolite in another material resistant to the temperature and other conditions employed in the process. Such matrix materials include synthetic or naturally occurring substances as well as inorganic materials such as clay, silica and/or metal oxides. The latter may be either naturally occuring or in the form of gelatinous precipitates or gels including mixtures of silica and metal oxides. Naturally occurring clays which can be composited with the zeolite include those of the montmorillonite and kaolin families, which families include the sub-bentonites and the kaolins commonly known as Dixie, McNamee, Georgia and Florida clays or others in which the main mineral constituent is hallosite, kaolinite, dickite, nacrite or anauxite. Such clays can be used in the raw state as originally mined or initially subjected to calcination, acid treatment or chemical modification.

In addition to the foregoing materials, the zeolites employed herein may be composited with a porous matrix material, such as alumina, silica-alumina, silica-magnesia, silica-zironica, silica-thoria, silica-berylia, silica-titania as well as ternary compositions, such as silica-alumina-thoria, silica-alumina-zironcia, silica-alumina-magnesia and silica-alumina-zironcia. The matrix may be in the form of cogel. The relative proportions of zeolite component and inorganic oxide gel matrix may vary widely with the zeolite content ranging from between about 1 to about 99 percent by weight and more usually in the range of about 5 to about 80 percent by weight of the composite.

The silica to alumina mole ratio of the base zeolite component may be determined by conventional analysis. This ratio is meant to represent, as closely as possible, the ratio in the rigid anionic framework of the zeolite crystal and to exclude aluminum in the binder or in cationic or other form within the channels. Although zeolites with a silica to alumina ratio of at least 12 are useful, it is preferred to use zeolites having higher ratios of at least about 30. Such zeolites after activation, e.g. heating for 1 hour at 540°C in an inert atmosphere, acquire an intracrystalline sorption capacity for normal hexane which is greater than that for water, i.e. they exhibit "hydrophobic" properties. This hydrophobicity property would indicate that a suitable medium for applying the silicon-containing compound used herein to the zeolite should be organic, e.g. n-hexane, benzene, toluene, chlorofrom, etc. and not water. If an organic medium were to be used, the silicon compound would be dissolved therein and the hydrophobic properties of the zeolite would not work against deposition of the silicon compound on the zeolite. However, it has been surprisingly found that an aqueous emulsion or dipersion of a silicon-containing compound is useful for this purpose.

The economic advantages accompanying the present method for catalyst preparation, when compared to preparing such a catalyst via contact with an organic solution of a silicon compound, are numerous. Water vapor can be vented to the atmosphere while organic vapor can not. The use of water in place of organic solvent, when a "suitable solvent" as required by the prior art for "dissolving" a particular compound which is not soluble in water, is not so readily apparent an economic advantage. Clearly, and unexpectedly, the use of silicon-containing water emulsion or dispersion in place of an organic solvent in treatment of a hydrophobic crystalline zeolite to produce even an equivalent product is an advantage which would not be readily apparent.

As noted hereinbefore, the present method of applying the silicon-containing compound provides a porous crystalline aluminosilicate zeolite which, upon subsequent thermal treatment, has a coating of silica deposited thereon. Such coating extensively covers the external surface of the zeolite and resides substantially completely on the external surface. The coating of silica is deposited on the surface of the zeolite by contacting the latter with the aqueous emulsion or dispersion of a silicone compound having a molecular size incapable of entering the pores of the zeolite and subsequently heating in an oxygen-containing atmosphere, such as air, to a temperature about 300°C but below a temperature at which the crystallinity of the zeolite is adversely affected at a rate such that the silicone compound does not volatize before undergoing oxidation of silica.

The silicon-containing, e.g., silicone, compound utilized to effect the silica coating of the zeolite component can be characterized by the general formula:

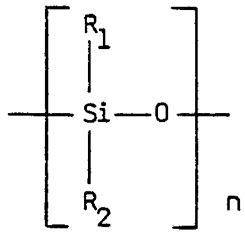

wherein $R_1$ is hydrogen, fluorine, hydroxy, alkyl, aralkyl, alkaryl or fluoro-alkyl. The hydrocarbon substituents generally contain from 1 to 10 carbon atoms and preferably are methyl or ethyl groups. $R_2$ is selected from the same group as $R_1$, or other than hydrogen and other than methyl if $R_1$ is hydrogen and n is an integer of at least 10 and generally in the range of 10 to 1000. The molecular weight of the silicone compound employed can generally be between about 500 and about 20,000 and preferably within the approximate range of 1,000 to 10,000. Representative silicone compounds include dimethylsilicone, diethylsilicone, phenylmethylsilicone, ethylhydrogensilicone, phenylhydrogensilicone, methylethylsilicone, phenylethylsilicone, diphenylsilicone, methyltrifluoropropylsilicone, ethyltrifluoropropylsilicone, polydimethylsilicone, tetrachlorophenylmethyl silicone, tetrachlorophenylethyl silicone, tetrachlorophenylhydrogen silicone, tetrachlorophenylphenyl silicone, methylvinylsilicone and ethylvinylsilicone.

The silicone compound as hereinbefore described is combined with water in any manner and amount such that an aqueous emulsion or dispersion of the silicone compound is formed. Preferably, the silicone compound will comprise from about 2 percent to 50 percent by weight of such an aqueous emulsion or dispersion, more preferably from about 5 percent to 15 percent by weight.

The aqueous emulsion or dispersion of the silicone compound is contacted with the above described zeolite at a temperature between about 10°C and about 200°C for a period of time sufficient to deposit the ultimately desired amount of silicone thereon. Time of contact will generally be within the range of 0.2 to 5 hours, during which time the mixture is desirably subjected to evaporation. The resulting residue is then calcined in an oxygen-containing atmosphere, preferably air and preferably at a rate of 0.2° to 5°C/minute to a temperature greater than 300°C but below a temperature at which the crystallinity of the zeolite is adversely affected. Generally, such temperature will be below 700°C. Preferably the temperature of calcination is within the approximate range of about 350°C to about 550°C. The product is maintained at the calcination temperature usually for 1 to 24 hours to yield a silica-coated zeolite containing between about 0.5 to about 30 weight percent and preferably between about 1 and 15 weight percent silica.

In is process aspects, the present invention relates to the use of the silica-modified zeolite materials as catalysts for the conversion of aromatic compounds. The charge stock used herein for the selective production of para-dialkyl substituted benzenes containing alkyl groups of 1 to 4 carbon atoms by contact, under conversion conditions, with a catalyst comprising the hereinbefore described zeolite composition includes a hydrocarbon precursor selected from the group consisting of monoalkyl-substituted benzenes having 1—4 carbon atoms in the alkyl substituent, such as toluene, ethyl benzene, propyl benzene or butyl benzene and a mixture of such precursor or benzene with an alkylating agent containing from 1 to 4 carbon atoms.

Typical of the processes contemplated herein are disproportionation of toluene to benzene and xylene, wherein the proportion of para-xylene obtained is greatly in excess of its normal equilibrium concentration. Such process is effectively carried out at a temperature of between about 400°C and about 700°C at a pressure between about 1 atmosphere (101.3 kPa) and about 100 atmospheres (10133 kPa) utilizing a weight hourly space velocity of between about 1 and about 50.

The use of mixed aromatics as feed is also feasible. For example, a mixture of ethylbenzene and toluene is converted selectively to a mixture rich in para-dialkyl substituted benzene such as p-diethylbenzene and p-ethyltoluene, the latter predominating at high toluene ethylbenzene ratios in the feed.

Reaction of feedstock benzene, toluene, ethylbenzene, propylbenzene or butylbenzene with an alkylating agent containing from 1 to 4 carbon atoms is also contemplated using catalysts containing the zeolite compositions described hereinbefore. Products of this reaction include mixtures rich in para-dialkyl substituted benzenes. Suitable alkylating agents include olefins, alcohols, alkyl haldies, ethers and sulfides having from 1 to 4 carbon atoms. Representative of such compounds are ethylene, propylene, butylene, methanol, ethanol, propanol, butanol, methyl chloride, ethyl chloride, propyl chloride, butyl chloride, dimethylether, dimethylsulfide, diethylether, diethylsulfide, dipropylether, dipropylsulfide, dibutylether, and dibutylsulfide. Akylation is suitably carried out at a temperature between about 250°C and about 700°C at a pressure between about 1 atmosphere (101.3 kPa) and about 100 atmospheres (10133 kPa) employing a weight hourly space velocity of between about 0.1 and about 200. The mole ratio of feedstock/alkylating agent may be from about 1/1 to about 20/1 for this reaction.

It is contemplated that the conversion process described herein may be carried out as a batch type, semicontinuous or continuous operation utilizing a fixed or moving bed catalyst system. The catalyst after use is conducted to a regeneration zone wherein coke is burned from the catalyst in an oxygen-containing atmosphere, e.g. air, at an elevated temperature, after which the regenerated catalyst is recycled to the

conversion zone for further contact with the charge stock. With use of the present silica-coated zeolite catalyst, regeneration has been found to restore the activity of the catalyst to a high level, thereby providing a long catalyst life. It is particularly feasible to conduct the desired conversion in the presence of hydrogen utilizing a hydrogen/hydrocarbon mole ratio of between about 2 and about 20, with hydrogen pressure extending from 1 atmosphere (101.3 kPa) up to 100 atmospheres (10133 kPa). The presence of hydrogen in the reaction zone has been found to very substantially reduce the aging rate of the catalyst.

While the above process has been described with reference to selective production of para-dimethyl substituted benzens, typified by para-xylene, it is contemplated that other para-dialkyl subsituted benzenes, wherein the alkyl group contains from 1 to 4 carbon atoms may similarly be selectively produced. Thus, uitlizing the technique described herein, it is contemplated that with selection of suitable precursor, a mixture of ethyl benzene and toluene may be selectively converted to para-ethyl toluene; ethyl benzene may be selectively converted to diethyl benzene, propyl benzene may be converted to dipropyl benzene and butyl benzene may be selectively converted to dibutylbenzenes.

The following examples will serve to illustrate the method, composition and conversion process of the present invention without limiting the same.

Example 1

To 2.0 grams of $NH_4ZSM$—5 of 1—2 μm crystal size was added 0.45 gram phenylmethylsilicone as a 9% emulsion in water. After distillation of the water the residue was program ari-calcined at 1°C/minute and then 7 hours at 538°C. The resulting catalyst contained a nominal 9% extracrystalline silica.

Example 2

A sample of extracyrstalline silica-modified HZSM—5 prepared as in Exmaple 1 was tested in a flow reactor for toluene disproportionation at 482°C, 6.5 WHSV, 400 psig (2859 kPa), and a hydrogen/toluene mole ratio of 4.0. The results are shown in Table I below and are compared to data from an intracrystalline-modified HZSM—5 catalyst prepared as in Example 1 but using an aqueous emulsion of methylhydrogen-silicone which can penetrate the pores of ZSM—5. The intracrystalline-modified HZSM—5 had 13% intra-crystalline silica.

TABLE I

Wt.% of Product Xylenes

| | p-Xylene | m-Xylene | o-Xylene |
|---|---|---|---|
| Thermodynamic Equlibrium | 24 | 52 | 24 |
| 13% Intracrystalline Silica | 25 | 52 | 23 |
| 9% Extracrystalline Silica | 35 | 48 | 17 |

The above results show a 45.8 percent increase in the valuable para-xylene isomer when compared to expected thermodynamic equilibrium results. The extracyrstalline-modified catalyst of the present invention also provided an improvement over the same process conducted over an intracyrstalline-modified catalyst of 40 perecent. The process conducted with the catalyst of the present invention futher provided a reduced metal-isomer, the least desirable of the xylene isomers.

Example 3

Alylation of toluene with methanol is carried out using the extracrystalline silica-modified HZSM—5 prepared as in Example 1. Operating temperatures are varied through the range of 400—500°C, while maintaining 10 WHSV, atmopsheric pressure (101.3 kPa), a toluene/methanol mole ratio of 4.0, and a hydrogen/toluene mole ratio of 2.0. Table II summarizes the results.

TABLE II

| Temperature °C | Wt.% p-Xylene in Product Xylenes |
|---|---|
| 550 | 88 |
| 500 | 91 |
| 450 | 94 |
| 400 | 95 |

Example 4

Alkylation of toluene with ethylene is carried out using the extracrystalline silica-modified HZSM—5 prepared as in Example 1. Operating temperatures are varied through the range of 400—425°C while maintaining 29 WHSV (toluene), 100 psig (791 kPa), and a toluene/ethylene/hydrogen mole ratio of 8/1/3. Table III sumarizes the results.

TABLE III

| Temperature °C | Wt.% p-Ethyltoluene in Product Ethyltoulenes |
|---|---|
| 400 | 91 |
| 425 | 94 |

It should be recognized that the advantage for catalyst preparation in accordance with the present invention include the fact that an aqeuous emulsion or dispersion of the silicone fluid may be substituted for an organic solution thereof in the preparation of the zeolite catalyst with economic advantages that on a commerical scale are quite significant. Additionally, it will be noted that as hereinbefore described the process water used in the silicone emulsion may be vented to the atmosphere whereas organic solvents such as hexane may not and must be condensed to avoid hydrocarbon emissions.

**Claims**

1. A method for preparing a zeolite composition especially useful as a catalyst for promoting selective conversion of aromatic feedstocks to conversion products rich in para-dialkyl substituted benzene compounds, which method comprises:

A) contacting a crystalline zeolite base component having an alpha activity of from 2 to 5000, a xylene sorption capacity greater than 1 gram xylene per 100 grams of zeolite and an ortho-xylene sorption time for 30 percent of said sorption capacity measured at 120°C and a xylene partial pressure of 600 ± 100 Pa of greater than 10 minutes with an aqueous emulsion or dispersion os a silicon-containing compund of a molecular size substantially incapable of entering the pores of the zeolite, said silicon-containing compound having the general formula:

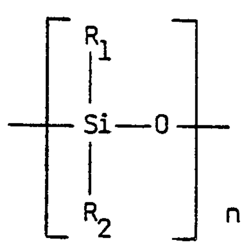

wherein $R_1$ is hydrogen, fluorine, hydroxy, alkyl, aralkyl, alkaryl or fluoro-alkyl, the hydrocarbon substituents containing from 1 to 10 carbon atoms, $R_2$ is selected from the same group as $R_1$, other than hydrogen, and other than methyl if $R_1$ is hydrogen, and n is an integer of at least 10, said contacting occurring at a temperature of from 10°C to 200°C for a time sufficient to permit from 0.5 weight percent to 30 weight percent silica to be deposited on the crystalline zeolite base component upon heating said base component in an oxygen-containing atmosphere at a temperature of from 300°C to 700°C, and

B) heating the crystalline zeolite base component, which has been contacted with said silicon-containing compound, in an oxygen-containing atmosphere at a temperature of from 300°C to 700°C to thereby provide said zeolite composition having deposited thereon a coating of silica which extensively covers and resides substantially exclusively on the external surface of said zeolite composition, said crystalline zeolite base component being characterized by a silica/alumina mole ratio of at least 12, a constraint index within the approximate range of 1 to 12 and, after heating at 540°C for 1 hour in an inert atmosphere, an intracrystalline sorption capacity for normal hexane which is greater than for water.

2. A method according to Claim 1 wherein said crystalline zeolite base component has the structure of ZSM—5, ZSM—5/ZSM—11 intermediate, ZSM—11, ZSM—12, ZSM—23, ZSM—35, ZSM—38 or ZSM—48.

3. A method according to Claim 1 or Claim 2 wherein said silicon-containing compound is selected from dimethylsilicone, diethylsilicone, phenylmethylsilicone, ethylhydrogensilicone, phenylhydrogensilicone, methylethylsilicone, phenylethylsilicone, diphenylsilicone, methyltrifluoropropylsilicone, ethyltrifluoropropylsilicone, polydimethylsilicone, tetrachlorophenylmethyl silicone, tetrachlorophenylethyl silicone, tetrachlorophenylhydrogen silicone, tetrachlorophenylphenyl silicone, methylvinylsilicone or ethylvinylsilicone.

4. A method according to any of Claims 1 to 3 wherein the heating of zeolite base component which has been contacted with silicon-containing compounds is carried out within the temperature range of 350°C to 550°C and takes place at the rate of from 0.2° to 5°C per minute.

5. A method according to any of Claims 1 to 4 wherein the zeolite base component comprises ZSM—5 and wherein the silicon-containing compound is phenylmethylsilicone or dimethylsilicone.

**Patentansprüche**

1. Verfahren zur Herstellung einer Zeolithzusammensetzung, die als Katalysator zur Förderung der selektiven Umwandlung aromatischer Ausgangsmaterialien zu Umwandlungsprodukten besonders vorteilhaft ist, die reich an p-dialkylsubstituierten Benzolverbindungen sind, wobei dieses Verfahren umfaßt;

A) Kontakt einer kristallinen Zeolithgrundkomponente mit einer Alpha-Aktivatäte von 2 bis 5000, einer Xylol-Sorptionskapazität von größer als 1 g Xylol pro 100 g Zeolith und einer Sorptionszeit für o-Xylol für 30% der Sorptionskapazität, bei 120°C und einem Partialdruck für Xylol von 600 ± 100 Pa gemessen, von größer als 10 Minuten, mit einer wässrigen Emulsion oder Dispersion einer siliziumhaltigen Verbindung mit einer Molekülgroße, die im wesentlichen in die Poren des Zeolith nicht eindringen kann, wobei diese siliziumhaltige Verbindung die allgemeine Formel aufweist:

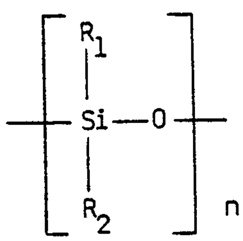

worin $R_1$ Wasserstoff, Fluor, eine Hydroxy-, Alkyl-, Aralkyl-, Alkaryl- oder FLuoralkylgruppe ist, die Kohlenwasserstoffsubstituenten von 1 bis 10 Kohlenstoffatome enthalten, $R_2$ aus der gleichen Gruppe wie $R_1$ ausgewählt ist, außer Wasserstoff und der Methylgruppe, wenn $R_1$ Wasserstoff ist, und n eine ganze Zahl von mindestens 10 ist, wobei dieser Kontakt bei einer Temperatur von 10°C bis 200°C während eines ausreichenden Zeitraumes erfolgt, damit von 0,5 Gew.-% bis 30 Gew.-% Siliziumdioxid beim Erwärmen der Grundkomponente in einer sauerstoffhaltigen Atmosphäre bei einer Tempeatur von 300°C bis 700°C auf der kristallinen zeolithgrundkomponente abgeschieden werden, und

B) erwärmen der kristallinen Zeolithgrundkomponente, die mit der siliziumhaltigen Verbindung in Kontakt gebracht wurde, in einer sauerstoffhaltigen Atmosphäre bei einer Temperatur von 300°C bis 700°C, um dadurch die Zeolithzusammensetzung zu schaffen auf der ein Siliziumdioxidüberzug abgeschieden ist, der die Außenoberfläche der Zeolithzusammensetzung umfassend abdeckt um im wesentlichen ausschließlich auf dieser liegt, wobei die kristalline Zeolithgrundkomponente durch ein Siliziumdioxid/Aluminiumoxyd-Molverhältnis von mindestens 12, einen Zwangsindex innerhalb des ungefähren Bereiches von 1 bis 12 und nach einem einstündigen Erwärmen bei 540°C in einer inerten Atmosphäre durch eine intrakristalline Sorptionskapazität für n-Hexan gekennzeichnet ist, die größer als die für Wasser ist.

2. Verfahren nach Anspurch 1, worin die kristalline Zeolithgrundkomponente die Struktur von ZSM—5, einer Zwischenstufe von ZSM—/ZSM—11, ZSM—11, ZSM—12, ZSM—23, ZSM—35, ZSM—38 oder ZSM—48 aufweist.

3. Verfahren nach Anspruch 1 oder Anspruch 2, worin die silizumhaltige Verbindung aus Dimethylsilan, Diethylsilan, Phenylmethylsilan, Ethylhydrogensilan, Phenylhydrogensilan, Methylethylsilan,

Phenylethylsilan, Diphenylsilan, Methyltrifluorpropylsilan, Ethyltrifluoroproylsilan, Polydimethylsilan, Tetrachlorphenylmethylsilan, Tetrachlorphenylethylsilan, Tetrachlorphenylhydrogensilan, Tetrachlorphenylphenlysilan, Methylvinylsilan oder Ethylvinylsilan ausgewählt ist.

4. Verfahren nach einem der Ansprüche 1 bis 3, worin das Erwärmen der Zeolithgrundkomponente, die mit der seliziumhaltigen Verbindung in Kontakt gebracht wurde, innerhalb des Temperaturbereichs von 350°C bis 550°C durchgeführt wird und bei einer Geschwindigkeit von 0,2°C bis 5°C pro Minute stafffindet.

5. Verfahren nach einem der Ansprüche 1 bis 4, worin die Zeolithgrundkomponent ZSM—5 umfaßt und die siliziumhaltige Verbindung Phenylmethylsilan oder Dimethylsilan ist.

## Revendications

1. Un procédé de préparation d'une composition zéoltique, notamment utile comme catalyseur pour favoriser la conversion sélective de charges aromatiques en produits de conversion riches en paradialkylbenzène, ce procédé consistant à:

A) mettre un composant à base de zéolite cristalline présentant une activité alpha comprise entre 2 et 5000, une capacité de sorption pour le xylène supérieure à 1 g de xylène par 100 g de zéolite et un temps de sorption d'orthoxylène pour 30% de cette capacité de sorption mesurée à 120°C et sous pression partielle de xylène égale a 600 ± 100 Pa, supérieure à 10 minutes, de contact d'une émulsion ou dispersion aqueuse d'un dérivé contenant du silicium dont les dimensions moléculaires le rendent sensiblement incapable de pénétrer dans les pores de la zéolite, ce dérivé contenant du silicium présentant la formula générale suivante:

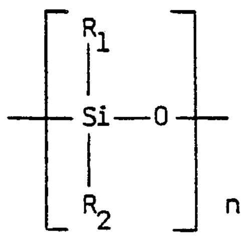

dans laquelle: $R_1$ représente un atome d'hydrogène, de fluor, un groupe hydroxy, un radical alkyle, aralkyle, alkaryle ou fluoroalkyle, les substituants hydrocarbonés contenant de 1 à 10 atomes de carbone; $R_2$ est choisi dans le même groupe que $R_1$, qui est différent de l'hydrogène et qui est différent du radical méthyle si $R_1$ représente un atome d'hydrogène; et n étant un nombre entier égal au moins à 10, cette mise au contact ayant lieu à une température comprise entre 10 et 200°C pendant un temps suffisant pour permettre le dépôt de 0,5% à 30% de silice sur le composant à base de zéolite cristalline par chauffage de ce composant de base sous une atmopshère contenant de l'oxygène à une température comprise entre 300 et 700°C; et

B) à chauffer le composant à base de zéolite cristalline qui a été mis au contact de ce dérive contenant du silicium, dans une atmopshère contenant de l'oxygène à une température comprise entre 300 et 700°C pour obtenir ainsi cette composition de zéolite sur laquelle est déposé un revêtement de silice qui la recouvre de façon extensive et se trouve concentré de façon sensiblement exclusive à la surface externe de cette composition de zéolite, ce composant à base de zéolite cristalline étant caractérisé par un rapport molaire silice/alumine au moins égal à 12, un indice de contrainte compris entre 1 et 12, et présentant après chauffage à 540°C pendant une heure dans une atmosphère inerte, une capacité de sorption intracristalline pour l'hexane normal supérieure à sa capacité de sorption pour l'eau.

2. Un procédé selon la revendication 1, dans lequel ce composant de base de zéolite cristalline présente la structure de la ZSM—5, de l'intermédiaire ZSM—5/ZSM—11, de la ZSM—11, ZSM—12, ZSM—23, ZSM—35, ZSM—38 ou ZSM—48.

3. Un procédé selon la revendication 1 ou la revendication 2, dans lequel ce dérivé contenant du silicium est choisi parmi les suivants: diméthylsilicone, diéthylsilicone, phénylméthylsilicone, éthylhydrogènesilicone, phénylhydrogènesilicone, méthyléthylsilicone, phényléthylsilicone, diphénylsilicone, méthyltrifluoropropylsilicone, éthyltrifluoropropylsilicone, polydiméthylsilicone, tétrachlorophénylméthylsilicone, tétrachlorophénylsilicone, tétrachlorophénylhydrogènsilicone, tétrachlorophénylphénylsilicone, méthylvinylsilicone et éthylvinylsilicone.

4. Une procédé selon l'une quelconque des revendications 1 à 3, dans lequel le chauffage du composant à base de zéolite qui a été mis au contact d'une dérivé contenant du silicium est mis en oeuvre dans un intervalle de tepérature compris entre 350 et 550°C, et a lieu à une vitesse de montée en température comprise entre 0,2° et 5°C par minute.

5. Un procédé selon l'une quelconque des revendications 1 à 4, dans lequel le composant à base de zéolite est consitué de ZSM—5 et le dérivé du silicium est le phenylméthylsilicone ou le diméthylsilicone.